# EUROPEAN PATENT APPLICATION

(11) **EP 0 616 803 A2**
(43) Date of publication of application: **28.09.1994**
(21) Application number: 94301155.1
(22) Date of filing: 17.02.1994
(51) Int. Cl.: A61K 31/095, A61K 31/195

(54) **Method for enhancing T-cell count**

(30) Priority: 25.02.1993 GB 9303854
(71) Applicant: Holt, John Alfred Gorton, Perth, Western Australia 6009 (AU)
(72) Inventor: Holt, John Alfred Gorton, Perth, Western Australia 6009 (AU)
(74) Representative: Brown, David Leslie

(57) **Abstract**

A method of enhancing T-cell count in an immunodeficient individual comprises administering to the individual a non-toxic organic disulfide of the type defined (e.g. cystine or oxidised glutathione) and while the disulfide is present in the bloodstream administering to the individual an effective dose of microwave electromagnetic radiation of frequency in the range of about 400-450 MHz, preferably in the range of about 432-436 MHz.

## Description

The present invention relates to a novel method for enhancing T-cell count in humans infected with the human immunodeficiency virus (HIV). European Patent Application No. 0531031 describes compositions and methods for cancer therapy. The present invention employs selected agents from that therapy in the novel application to enhancement of T-cell count.

The progression of HIV in an infected individual is generally accompanied by a fall-off in the blood count of a certain thymus-produced lymphocyte known as a T₄-cell or CD₄-cell. T₄ levels in the blood can typically fall from about 600-1700 per mm ³ (normal) to about 300 or less per mm³ in an individual with advanced HIV progression. The T₄ count of an individual is thus indicative of the level of immunologically competent or potentially competent defender cells circulating in the individual's bloodstream. It is believed that the HIV virus actually replicates within the infected individual's T₄-cells, which thereby release more HIV viruses into the bloodstream.

The present invention is based on my surprising finding that, by administering to an immunodeficient, e.g. HIV-infected, individual an effective amount of one or more selected chemical agents and while said agent(s) is/are present in the bloodstream of the individual administering to the individual an effective dose of microwave electromagnetic radiation of frequency in the range of about 400-450 MHz, the fall-off in the individual's T₄-cell count can be halted or reversed, leading to a greater ability of treated individuals to resist the secondary infections that accompany advanced HIV infection (the so-called "full-blown" AIDS).

More particularly, the chemical agent(s) used should be selected from non-toxic organic disulfides. Included within the scope of the invention is the use of non-toxic precursors of the said agents, which are administered to the patient and are modified *in vivo* to provide the desired agent(s) in the bloodstream. For example, a thiol can be administered simultaneously with an oxidising agent to provide the corresponding disulfide in the bloodstream.

According to a first aspect, therefore, the invention provides a method for enhancing T-cell count in an immunodeficient individual, which comprises administering to the individual an effective amount of a non-toxic organic disulfide of the general formula

R - S - S - R (I)

in which R is an ethyl group β,β-disubstituted by two substituents selected from amino, carboxy, carboxy-(C₁₋₄ alkyl)carbamoyl, C₂₋₅ alkanamido substituted by amino and carboxy, and salt derivatives thereof, and while said organic disulfide is present in the bloodstream of the individual administering to the individual an effective dose of microwave electromagnetic radiation of frequency in the range of about 400-450 MHz.

According to a second aspect, the invention provides the use of a non-toxic organic disulfide of formula I in the preparation of a composition or compositions for use in a method for enhancing T-cell count in an immunodeficient individual according to the first aspect of the invention as described above.

The expression "non-toxic" used herein refers to an acceptably low level of toxicity when a compound is present in an effective amount, and not necessarily to a complete absence of toxic effects. In particular, compounds which have only a temporary toxic effect and do no significant permanent harm, will be referred to and understood as "non-toxic" herein.

The substituents of the ethyl group of R in formula I are preferably different from each other. Any C₂₋₅ alkanamido group is preferably terminally disubstituted by one amino and one carboxy group or salt derivatives thereof.

A particularly preferred disulfide is cystine, which has the formula: 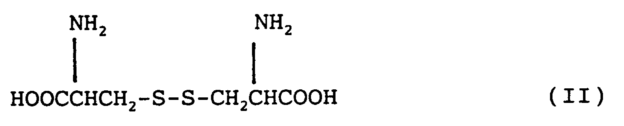
or a non-toxic salt thereof.

An optically active form of cystine, or alternatively an optically inactive (internally compensated) form or a racemic mixture, may be used. The laevorotatory form L-cystine is preferred by reason of its availability.

There may also be mentioned as a suitable organic disulfide oxidised glutathione (N,N'-[dithiobis[1-[(carboxymethyl)carbamoyl]-ethylene]]diglutamine),which has the formula: 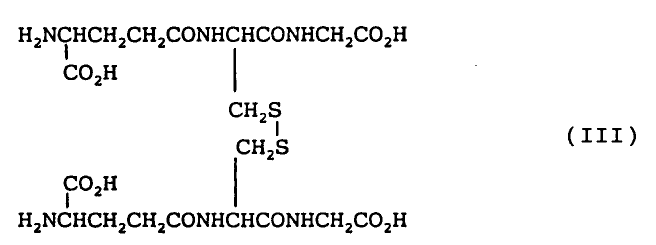
or a non-toxic salt thereof. Any optically active or optically inactive form of oxidised glutathione, including a racemic mixture, may be used.

Mixtures of different disulfides of formula I may be used, if desired, or different disulfides may be administered one after another in rapid succession. It is most preferred to use a mixture or sequential administration of cystine and oxidised glutathione.

The organic disulfide of formula I may optionally be used in association with a potentiating agent which comprises one or more oxidising agent selected from organic peroxides and hydroperoxides of the general formulae

R¹ - O - O - R² (IV)

and

R³ - O - OH (V)

respectively, in which R¹, R² and R³, which may be the same or different, are organic groups, most preferably selected from alkyl (e.g. methyl, ethyl, propyl, n-butyl, s-butyl or t-butyl) groups which may optionally be mono- or poly-substituted.

In the oxidising agents of formulae IV and V as defined above, substituents of alkyl groups, when present, may be selected from any substituent atom(s) and group(s) whose presence as substituent does not significantly reduce the effectiveness of the agents in the therapy described and does not lead to intolerable side effects such as toxicity.

Examples of suitable substituents of R, R² and R³ include halo (e.g. fluoro, chloro and bromo), nitro, aryl (e.g. phenyl) and substituted aryl (e.g. aryl mono- or poly-substituted by alkyl, halo and/or nitro). Preferred substituents, when present, are aryl groups.

A particularly preferred oxidising agent is t-butyl hydroperoxide, although cumene hydroperoxide may also find use in certain treatment methods.

Without wishing to be bound by theory, it is believed that the potentiating agent acts to prevent degradation of the disulfide active agents to corresponding thiols in the patient's bloodstream.

Trials have shown that the method of the present invention can be effective in boosting the T₄-cell count of an immunodeficient individual to levels approaching normality, or within the normal range, which can result in the patient feeling clinically well and to a large extent resistant to many of the infections which could otherwise take place. The present invention therefore has applicability alongside other clinical procedures which are aimed at combating the HIV virus itself but which do not themselves substantially or permanently halt or reverse the fall-off in T₄-cell count as the HIV infection advances.

The composition form(s) by which the active and, if present, potentiating agent(s) is or are administered to the patient may take any suitable form and employ any suitable conventional pharmaceutical carrier(s) or excipient(s). Parenteral administration of active agents and, if present, potentiating agents, is convenient. The composition form is preferably an aqueous solution or suspension suitable for intravenous drip, infusion or injection. Intravenous drip, infusion or injection is the preferred administration route by virtue of its rapid delivery of the active agent to the bloodstream with minimal degradation of the active agent. Topical administration as a paint to the patient's skin may however be more convenient for some patients.

The composition may if desired include additional components such as salts (e.g. sodium chloride), solubilising and/or suspending agents and the like. The compositions are prepared by standard mixing techniques that will be readily apparent to those skilled in this art. In the case of sparingly soluble active agents a suitable solubilising agent should first be dissolved in the aqueous medium.

The dose of chemical agent is selected according to the body weight of the patient, the tolerance of the patient to the active agent used, the ability of the active agent to remain in the bloodstream without loss or degradation, the efficacy of the particular agent, the T₄-cell count of the patient to be treated, and the projected length of the overall treatment program.

An aqueous solution of L-cystine at a concentration of up to approximately 20 g/l (e.g. from about 10 to 20 g/l, suitably about 20 g/l) is convenient, and in the case of oxidised glutathione an aqueous solution at a concentration of up to about 40 g/l (e.g. from about 20 to about 40 g/l, suitably about 40 g/l) is convenient. This enables delivery of a suitable daily dose of L-cystine of about 40 mg per kg body weight and a suitable daily dose of oxidised glutathione of about 20 mg per kg body weight.

To assist the passing of L-cystine into solution, a solubilising agent such as N-methyl-D-glucamine is also conveniently present in the solution. A small amount of an oxidant such as t-butyl hydroperoxide may be present to prevent the L-cystine or oxidised glutathione from degrading to its respective thiol cysteine or glutathione prior to administration.

The following solutions have been found to be particularly suitable:

### L-cystine

An aqueous solution of L-cystine is prepared by first dissolving 70 g of N-methyl-D-glucamine in 1 litre of normal (0.9%) saline and then dissolving in 20 g of L-cystine. 3 ml of a 0.25% aqueous solution of t-butyl hydroperoxide per litre is then added, to prevent reduction of the disulfide during storage.

### Oxidised glutathione

An aqueous solution of oxidised glutathione is prepared by dissolving 40 g of oxidised glutathione in 1 litre of normal (0.9%) saline. 3 ml of a 0.25% aqueous solution of t-butyl hydroperoxide per litre is then added, to prevent reduction of the disulfide during storage.

### T-butyl hydroperoxide (potentiating agent)

An aqueous solution of t-butyl hydroperoxide is prepared by diluting 3.5 ml of 70% t-butyl hydroperoxide in 1 litre of normal (0.9%) saline.

### Cumene hydroperoxide (potentiating agent)

An aqueous solution of cumene hydroperoxide is prepared by diluting 2.5 ml of an 80% solution of cumene hydroperoxide in cumene in 1 litre of normal (0.9%) saline.

Doses of L-cystine above about 2.5g per day produce a progressively greater risk of nausea in the patient, so that a preliminary test of the patient's tolerance may be advisable at such higher doses.

In the case of a potentiating agent and organic disulfide agent used in association with each other, the agents may be administered simultaneously or sequentially. It is preferred that the potentiating agent is administered first by intravenous drip or infusion or slow injection, followed immediately by the active agent(s).

After administration to the patient of the active, and optionally potentiating, agent(s) according to the invention, a period of time should pass to allow the active agent(s) to circulate in the bloodstream, whereupon the microwave radiation should be administered without undue delay. The radiation should normally be begun to be administered between 1 and 15 minutes after administration of the active agent(s) to the patient.

To administer the microwave radiation, which suitably covers the entire body (or at least a major part of the body) of the patient, the patient is brought close to a suitable number of microwave antennae (e.g. 2 to 4 antennae arranged around the patient's body) and the antennae energised to transmit UHF microwave radiation. For this purpose the patient may conveniently be moved under the antennae.

The total antennae power should be less than about 4 kW (e.g. adjustable between about 0.4 and about 3 kW) in order to remain tolerable to the average patient. Patients may preferably be treated in two or more sessions, preferably at least 7, most preferably between 15 and 25 sessions, at daily intervals or every other or every third day for a treatment period of up to about four weeks. Preferably, each session day involves administration of the active agent followed quickly by from 1 to about 5 exposures (e.g. three exposures) to the microwave radiation, each exposure to the radiation lasting from about 30 seconds to 3 minutes, with a 5 to 40 minute rest period between exposures. The total length of treatment on each session day will be dependent on the length of time over which the agent(s) remain in the bloodstream without substantial loss of activity, usually less than 40 minutes.

The first exposure to the microwaves should not exceed 800 Watt-minutes in total. This should preferably be followed by a resting time between 10 and 20 minutes. The second and subsequent exposures should preferably be made at time intervals between 5 and 10 minutes, and at the same radiation dose (i.e. maximum 800 Watt-minutes). On each day the total applied energy should not exceed about 3,200 Watt-minutes. On the first occasion of treatment for a patient not previously exposed to microwaves the energy limits recommended above should be particularly carefully observed. However, the second and/or third and/or subsequent exposures to the microwaves can employ doubled or quadrupled total energy output from the antennae. If too high a power is used, however, the patient's body temperature will rise, with possibly dangerous consequences.

As mentioned above, the microwave radiation should be in the frequency range of about 400-450 MHz. More preferably, the frequency should be in the range of about 425-440 MHz, most preferably 432-436 MHz (e.g. about 434 MHz).

The antennae are suitably standard commercially available microwave antennae of a convenient size and shape to accommodate the shape of the patient's body. The antennae preferably be provided with adjustable inductor coils in known manner to emit predominantly H-wave microwave radiation of wavelength between about 65 and 75 cm (most preferably about 69 cm). Each antenna may consist of a single circular turn approximately 19.5 cm in diameter (the diameter being chosen to resonate at the frequency of the transmitter), the plane of the circular antenna being held substantially tangential to the curvature of the patient's body. Such a circular antenna will emit substantially H-wave radiation towards the patient's body. While the presence of E-wave radiation will not necessarily hinder the efficacy of the radiation, it can lead to greater heating of the patient's skin, which is undesirable.

Each antenna is suitably connected to a UHF generator by a coaxial cable via circulators to prevent adverse interaction between antennae. Each generator may if desired be set at a slightly different frequency at or around a central frequency in the range already mentioned (preferably about 434 MHz).

One or two pairs of such circular (loop) antennae may suitably be used, the antennae of a pair being suitably positioned on a common axis (the axes in the case of two pairs being suitably orthogonal to each other).

All equipment within about 2 metres of the antennae should be of non-metallic materials such as wood or plastic, including for example the patient support table. If regulations require this frequency radiation to be shielded to avoid interference with other, standard, frequencies in a particular country or region, then a Faraday cage must enclose the patient. The Faraday cage should have a minimum internal dimension of 5m long, 2m wide and 2m high, with the couch mounted so that the patient is in the approximate centre of this volume.

A particularly preferred treatment regime is as follows: Four loop antennae are arranged around the patient's body. An infusion of t-butyl hydroperoxide (3 ml per kg body weight of a solution prepared by diluting 3.5 ml of 70% t-butyl hydroperoxide in one litre of normal (0.9%) saline) is slowly administered intravenously over a period of 10 minutes, followed immediately by intravenous cystine solution (40 mg per kg body weight) and oxidised glutathione solution (20 mg per kg body weight). The antennae are energised to 0.1 kW each (total power 0.4 kW) for patients of 50 ± 10 kg body weight, and increased proportionately for increased body weight of the patient being treated, to emit 434 MHz microwave radiation into the gap between the antennae, and the patient's entire body from the feet to above the vertex of the skull is slid slowly through the gap between the antennae over a period of about one minute. After a 15-20 minute rest period the patient is moved back through the gap between the antennae under the same antennae power. If it is desired to double the microwave dose at the second exposure to microwaves, the traverse of the patient can conveniently be reversed immediately to complete another pass between the antennae. (Alternatively, one transit could be made at double the previous antennae power). A second rest period, suitably about 5-10 minutes long, should then follow. The third and final phase would then comprise one to four transits through the gap between the antennae (or alternatively one transit at four times the power). The exact extent of radiation dosage on second and subsequent treatments on each day can readily be judged by a skilled worker by observing the response of the patient during previous treatment sessions. Weekly or more frequent monitoring of the patient's blood T₄-cell count should indicate a trend towards the normal range. The microwave dosage could be modified up or down in later treatments to reinforce the improvement, based upon observations of the treatment and response.

As described in my European Patent Application No. 0531031, the active agent according to the present invention exhibits anticancer properties in the presence of 400-450 MHz microwave electromagnetic radiation. As stated in the prior application, I believe that the active agent interferes with chemical reactions at the cancer site and may therefore be consumed at that site. The presence of cancer sites in the HIV-infected individual may therefore decrease the effectiveness of the present T₄-cell enhancement method, perhaps even to an extent that cannot be countered by an increased dose of active agent if the limit of tolerance of the patient to the active agent is thereby reached. In patients with both HIV and cancer, the use of a potentiating agent is therefore highly desirable.

Tests have yielded the following data, which are included purely for ease of understanding of the present invention, and not for limitation of the scope of the invention:

### EXAMPLE

### Compositions and administration volumes

An aqueous solution of L-cystine is prepared by first dissolving 58 g of N-methyl-D-glucamine in 1 litre of normal (0.9%) saline and then dissolving in 16 g of L-cystine. In the third treatment described below, 60 ml of the solution is administered via intravenous drip (delivering an approximately 1 g dose of cystine). In the fourth treatment described below the composition is modified so as to contain additionally approximately 4.lg penicillamine disulfide (which in some patients may exhibit a mildly potentiating effect but has a tendency to produce an allergic reaction which limits its general utility as a potentiating agent) per litre and 120ml of the solution is administered via intravenous drip (delivering an approximately 2g dose of cystine and 0.5g of penicillamine disulfide). 3 ml of a 0.25% aqueous solution of t-butyl hydroperoxide per litre is suitably added to prevent reduction of the disulfides.

### Toxicity

No nausea after the i.v. injection. No immediate or late changes in blood pressure, renal or liver function, electrolytes or haematology have been seen.

### Contra-indications

Cancerous tissue in the patient will reduce the degree of T₄-cell enhancement, with the result that the dosage of disulfide may need to be increased, possibly to the level of tolerance of the patient (at which point the administrable dose would cease to be effective).

### Case Histories

Three case histories will now be described, purely by way of illustration and example and for ease of understanding the present invention, but without limitation.

### CASE HISTORY A

PH, a male then aged about 39, was diagnosed as having HIV infection. No other disease present has been discovered.

**First Treatment** was a course of AZT for six months, which was terminated because of falling T₄-cell count at that time. About three years after diagnosis his T₄-cell count was 609 per mm³ and still falling slowly.

**Second Treatment** about one month after the T₄-cell count of 609 was taken was a course of Interferon followed by Alpha Interferon Inducer (a drug called Equimod). The Interferon induced partial liver failure with abnormalities in his liver function tests, but these returned to normal two months later. At about this time, his T₄-cell count was 486 per mm³.

**Third Treatment**, according to the present invention, consisted of seven treatment sessions over a period of 9 days commencing about one month after the T₄-cell count of 486. Each session consisted of intravenous injection of 1.0g cystine suspended in N-methyl-D-glucamine solution as described above, followed by three whole-body treatments with 434 MHz scanning from the vertex of the skull to the knees. Each whole-body treatment was according to the regime previously specified (800 Watt-minutes maximum), followed by a rest period of up to 20 minutes, then a further treatment as before (not exceeding 1600 Watt-minutes), a further rest period of 10 minutes and then a final treatment (not exceeding 3200 Watt-minutes) as before. Fourteen days after completion of the treatment program the patient's T₄-cell count peaked at 837 per mm³ and then slowly declined over the next few months to a count of 550 just under three months after the treatment.

**Fourth Treatment** was also according to the present invention and consisted of 14 treatment sessions over a period of 18 days commencing about three and a half months after completion of the third treatment described above. Under the fourth treatment regimen, he received for each session an intravenous injection of 2.0g cystine solution as described above together with 0.5g penicillamine disulfide. By 27 days after completion of the treatment program the patient's T₄-cell count was 650 and by 73 days after completion of the treatment program the count was 1090. Approximately 90 days after completion of the program the patient's T₄-cell count was 850. The patient's general health has remained satisfactory and apart from suffering on eye infection he has remained clinically well.

### CASE HISTORY B

A male then aged about 61 was diagnosed as having HIV infection, possibly from a transfusion about three years previously. Kaposi's Sarcoma was present on feet, legs and face and the local lesions were initially treated with a combination of microwaves and superficial X-ray therapy.

Regression of the cutaneous lesions was observed, but this was accompanied by a continuous decline in T₄ count, from a level of 245 at the time of treatment to 170 eight months later.

Four courses of treatment according to the present invention were then performed, using a combination of 434 MHz microwaves and cystine, oxidised glutathione and t-butyl hydroperoxide, over a period of one year. Two months after final course of treatment the patient's T₄ count was 916 (normal), and two years after that had fallen slightly, to 860 (normal).

At last contact the patient was well and asymptomatic and the Kaposi's Sarcoma was reduced to a few indolent scars at the site of some of the lesions on the feet, legs and face.

### CASE HISTORY C

A male then aged about 31 was diagnosed HIV positive.

Over the next four years the patient's T₄ count declined from 778 (normal) to 314.

A course of treatment according to the present invention was then performed, using a combination of 434 MHz microwaves and cystine and oxidised glutathione over a period of 12 days. The patient's T₄ count recovered to 745 (normal). About one year later the T-cell count had fallen again to 255, and was then raised to 421 by a further course of treatment using cystine only with microwaves. Six months later it had again fallen to 258 and was again raised, to 416, by a further course of treatment using cystine only with microwaves.

One year later the patient began a course of AZT treatment and his T-cell count fell to 200 over the first year of the AZT treatment.

A further treatment according to the present invention was then performed, using a combination of 434 MHz microwaves and cystine, oxidised glutathione and t-butyl hydroperoxide, and one year later his T₄ count had recovered to 745 (normal).

At last contact the patient was in good health.

## Claims

1. Use of a non-toxic organic disulfide of the general formula
R - S **-** S **-** R (I)
in which R is an ethyl group β,β-disubstituted by two substituents selected from amino, carboxy, carboxy-(C₁₋₄ alkyl)carbamoyl, C₂₋₅ alkanamido substituted by amino and carboxy, and salt derivatives thereof, in the preparation of a composition or compositions for use in a method for enhancing T-cell count in an immunodeficient individual in which method there is administered to the individual an effective amount of said non-toxic disulfide, and while said disulfide is present in the bloodstream of the individual there is administered to the individual an effective dose of microwave electromagnetic radiation of frequency in the range of about 400-450MHz.

2. A use according to claim 1, wherein the substituents of the ethyl group of R in formula I are different from each other.

3. A use according to claim 1 or 2, wherein any C₂₋₅ alkanamido group present is terminally disubstituted by one amino and one carboxy group or salt derivatives thereof.

4. A use according to claim 1 or 2, wherein the non-toxic organic disulfide is cystine or a non-toxic salt thereof.

5. A use according to claim 5, wherein the cystine or salt thereof is in an aqueous solution at a concentration of up to approximately 20 g/l (e.g. approximately 10-20 g/l).

6. A use according to any one of claims 1 to 3, wherein the non-toxic organic disulfide is oxidised glutathione or a non-toxic salt thereof.

7. A use according to claim 7, wherein the oxidised glutathione or salt thereof is in an aqueous solution at a concentration of up to approximately 40 g/l (e.g. approximately 20-40 g/l).

8. A use according to any one of the preceding claims, wherein more than one non-toxic organic disulfide of formula I is administered sequentially or simultaneously.

9. A use according to any one of the preceding claims, wherein the non-toxic organic disulfide(s) of formula I is/are used in association with a potentiating agent comprising one or more oxidising agent selected from organic peroxides and hydroperoxides of the general formula
R¹ - O - O - R² (IV)
and
R³ - O - OH (V)
respectively, in which R¹, R² and R³, which may be the same or different, are organic groups which may optionally be mono- or poly-substituted.

10. A use according to claim 9, wherein R¹, R² and/or R³ are selected from alkyl groups, and substituents thereof, when present, are selected from halo, nitro, aryl and substituted aryl.

11. A use according to claim 9 or 10, wherein the non-toxic organic disulfides cystine or a salt thereof and oxidised glutathione or a salt thereof are used and the potentiating agent is t-butyl hydroperoxide.

12. A use according to any one of claims 9 to 11, wherein the potentiating agent is administered separately from the organic disulfide(s) of formula I.
